# EUROPÄISCHE PATENTSCHRIFT

(11) **EP 2 744 433 B1**
(45) Veröffentlichungstag und Bekanntmachung des Hinweises auf die Patenterteilung: **30.03.2016**
(21) Anmeldenummer: 12746306.5
(22) Anmeldetag: 27.07.2012
(51) Int. Cl.: A61B 17/88, A61B 17/72

(54) **Medizinische Vorrichtung zur Knochenexpansion**
Medical device for bone expansion
Dispositif médical d'expansion osseuse

(30) Priorität: 18.08.2011 DE 102011110995; 11.05.2012 DE 102012207968
(43) Veröffentlichungstag der Anmeldung: 25.06.2014
(73) Patentinhaber: Militz, Matthias, 82418 Murnau (DE); Oehlbauer, Markus, 82418 Murnau (DE)
(72) Erfinder: Militz, Matthias, 82418 Murnau (DE); Oehlbauer, Markus, 82418 Murnau (DE)
(74) Vertreter: Kilian Kilian & Partner
(86) Internationale Anmeldenummer: PCT/EP2012/064838
(87) Internationale Veröffentlichungsnummer: WO 2013/023898

(56) Entgegenhaltungen:
- FR-A1- 2 653 006
- US-A1- 2005 209 629
- US-A1- 2009 005 782
- US-A1- 2009 182 336
- US-A1- 2011 077 651

## Beschreibung

Die vorliegende Erfindung betrifft eine medizinische Vorrichtung, die eine Expansionsvorrichtung zur Knochenexpansion aufweist.

Im Stand der Technik sind in der Medizin allgemein Verfahren und entsprechende Vorrichtungen zur Behandlung von Defekten an Röhrenknochen bekannt.

Beispielsweise kann die Behandlung von Infektionen von Röhrenknochen zu Defekten der Röhrenknochen führen, die nicht selten mehrere Zentimeter betragen. Das Standardverfahren zur Rekonstruktion des fehlenden Röhrenknochens bzw. zur Behebung des Defektes stellt die Kallusdistraktion dar.

Bei diesem Verfahren werden beide defektseitigen Enden des Röhrenknochens mittels eines Fixateurs derart gehalten, dass sich die defektseitigen Enden des Röhrenknochens gegenseitig berühren. Nach einer gewissen Zeit bildet sich zwischen den defektseitigen Enden des Röhrenknochens neues Knochengewebe, der sogenannte Kallus.

Der sich gebildete Kallus wird anschließend über den Fixateur mit bis zu max. 1 mm pro Tag so lange distrahiert, bis der Kallus den zu überbrückenden Defekt des Röhrenknochens bzw. den fehlenden Röhrenknochen ersetzt. Nach der Konsolidierung des distrahierten Kallus, d.h. der Verknöcherung des neu gebildeten Knochengewebes, kann der Knochen wieder belastet werden.

Wie hieraus ersichtlich wird, ist das Verfahren der Kallusdistraktion in Abhängigkeit von der Größe des zu behebenden Defekts langwierig und stellt eine erhebliche Belastung für den Patienten dar.

Außerdem ist das bekannte Verfahren der Kallusdistraktion mit erheblichen technischen Maßnahmen verbunden, welche aus der Notwendigkeit des Tragens des Fixateurs über einen langen Zeitraum resultieren. Der Fixateur muss einerseits an dem defekten Röhrenknochen befestigt werden und andererseits von außen für die Aufbringung der Zugkraft zugänglich sein. Nicht selten kommt es hierbei zu implantatbedingten Komplikationen, wie beispielsweise von Weichteilirritationen, die daraus resultieren, dass das den Röhrenknochen bedeckende Gewebe zwangsläufig durch den Fixateur bei der Kallusdistraktion gespannt wird.

Es ist Aufgabe der vorliegenden Erfindung, eine Vorrichtung zur Verfügung zu stellen, die eine schnelle und einfache Rekonstruktion/Behandlung eines Röhrenknochens ermöglicht.

Diese Aufgabe wird mit einer medizinischen Vorrichtung gemäß Anspruch 1 gelöst.

Grundgedanke der Erfindung ist es, einen Defekt eines Röhrenknochens dadurch zu beheben, dass ein Röhrenknochen, den ein Patient aus anatomischen Gründen nicht notwendigerweise benötigt, beispielsweise das Wadenbein (Fibula), in Längsrichtung gespalten (Osteotomie) und anschließend nach Bildung des Kallus an dem Spalt ausgedehnt wird.

Hierfür wird eine erfindungsgemäße medizinische Vorrichtung aufweisend eine Expansionsvorrichtung zur Knochenexpansion vorgeschlagen, die ein proximales Ende und ein distales Ende aufweist, zwischen denen sich die Expansionsvorrichtung in einer Längsrichtung erstreckt. Die Expansionsvorrichtung umfasst erfindungsgemäß weiterhin Ausdehnungseinrichtungen, die in der Längsrichtung aufeinanderfolgend angeordnet sind und zur Kraftausübung auf einen Knochen jeweils radial ausgedehnt werden können.

Die Expansionsvorrichtung ist zwischen dem proximalen Ende und dem distalen Ende derart dimensioniert, dass sie in einen auszudehnenden Röhrenknochen eingeführt werden kann.

Die erfindungsgemäße Expansionsvorrichtung wird bestimmungsgemäß in einen in Längsrichtung gespaltenen Röhrenknochen (Längsosteotomie) eingeführt und nach Ausbildung des Kallus an dem Längsspalt durch Ausdehnung der Expansionsvorrichtung bzw. der Ausdehnungseinrichtungen entlang ihrer Längsrichtung derart ausgedehnt, dass ein im Querschnitt größerer Röhrenknochen im Ergebnis erhalten wird.

Dadurch dass die Expansionsvorrichtung mehrere Ausdehnungseinrichtungen aufweist, können auf Teilabschnitte des Röhrenknochens, an denen sich die entsprechenden Ausdehnungseinrichtungen befinden, gleiche oder unterschiedliche Kräfte ausgeübt werden, wodurch der Röhrenknochen unabhängig von seinen in Längsrichtung vorliegenden Wandstärken und Stabilitätseigenschaften in einer bestimmten Weise ausgedehnt werden kann.

Anders ausgedrückt, kann durch die erfindungsgemäße Expansionsvorrichtung ein Röhrenknochen mit gewünschten Dimensionen erhalten werden. Beispielsweise können die Ausdehnungseinrichtungen derart ausgedehnt werden, dass sie sich einheitlich und gleichmäßig ausdehnen, d.h. dass der Durchmesser des auszudehnenden Röhrenknochens sich über die gesamte Länge des Röhrenknochens gleichmäßig vergrößert. In Abhängigkeit von Wandstärken und Stabilitätseigenschaften des Röhrenknochens kann es hierbei notwendig sein, dass auf die Teilabschnitte des Röhrenknochens über die Ausdehnungseinrichtungen unterschiedliche Kräfte aufgebracht werden.

Allerdings können die Ausdehnungseinrichtungen auch unterschiedlich ausgedehnt werden, d.h. nicht einheitlich und nicht gleichmäßig. Dies ist beispielsweise dann erforderlich, wenn aus einem Röhrenknochen konstanten Durchmessers ein Röhrenknochen mit nicht konstantem Durchmesser erhalten werden soll.

Die erfindungsgemäße Expansionsvorrichtung ist bevorzugt auch derart ausgestaltet, dass die Ausdehnungseinrichtungen unabhängig voneinander ausgedehnt werden können.

In einer bevorzugten Ausgestaltung der erfindungsgemäßen Expansionsvorrichtung weist diese beispielsweise 2, 3, 4, 5, 6, 7, 8, 9, 10, 11, 12, 13, 14, 15, 16, 17, 18, 19, 20, 21, 22, 23, 24, 25, 26, 27, 28, 29 oder 30 Ausdehnungseinrichtungen auf.

Die Ausdehnungseinrichtungen der erfindungsgemäßen Expansionsvorrichtung können gleich ausgebildet sein, beispielsweise dieselbe Größe aufweisen, und entlang der Längsrichtung der Expansionsvorrichtung entweder gleichmäßig oder ungleichmäßig angeordnet sein.

Die erfindungsgemäße Expansionsvorrichtung kann auch derart ausgebildet sein, dass die Ausdehnungseinrichtungen unterschiedlich ausgebildet, beispielsweise unterschiedliche Größen aufweisen, und entlang der Längsrichtung der Expansionsvorrichtung entweder gleichmäßig oder ungleichmäßig angeordnet sind, d.h. die Abstände zwischen den Ausdehnungseinrichtungen können entlang der Längsrichtung entweder gleich oder unterschiedlich sein.

In Abhängigkeit davon, wie die Ausdehnungseinrichtungen ausgebildet und entlang der Längsrichtung entweder gleichmäßig oder ungleichmäßig angeordnet sind, kann die erfindungsgemäße Expansionsvorrichtung beispielsweise an verschiedenartige Röhrenknochen angepasst werden.

In einer bevorzugten Ausgestaltung der erfindungsgemäßen Expansionsvorrichtung sind die Ausdehnungseinrichtungen in einem Hüllelement aufgenommen, das sich zwischen dem proximalen und distalen Ende der Expansionsvorrichtung in der Längsrichtung erstreckt.

Bevorzugte Materialien für das Hüllelement sind beispielsweise ein polyolefin-basiertes Elastomer, ein für medizinische Anwendungen geeignetes Silikon oder allgemein ein für medizinische Anwendungen geeignetes elastisches Polymer.

Vorteilhaft an dem Hüllelement ist, dass es die Ausdehnungseinrichtungen aufnimmt und bevorzugt abgedichtet einschließt und aus diesem Grund lediglich das Hüllelement aus einem für die Implantation geeigneten Material gebildet sein kann. Das heißt, die Ausdehnungseinrichtungen kommen während der Knochenexpansion bzw. der Kallusdistraktion nicht in direkten Kontakt mit dem Knochen oder Knochenmark, weshalb die Ausdehnungseinrichtungen lediglich im Hinblick auf die zu erwartenden Drücke und Kräfte materialmäßig ausgestaltet werden können.

Die Ausdehnungseinrichtungen können beispielsweise Kammern sein, die unabhängig voneinander mit einem Medium, wie beispielsweise einer Flüssigkeit, einem Gel oder einem Gas, gefüllt werden können.

Die Kammern sind beispielsweise ellipsoid- oder zylinderförmig ausgebildet.

In Abhängigkeit von der Anzahl der Kammern ist es möglich, Kräfte abschnittsweise auf den Röhrenknochen zur radialen Ausdehnung des Röhrenknochens auszuüben.

Beispielsweise sind die Kammern durch Ballons ausgebildet, die unabhängig voneinander mit dem Medium gefüllt werden können. Bevorzugt sind die Ballons aus einem elastischen Material, wie beispielsweise einem Elastomer, Silikon, Latex oder allgemein aus einem elastischen Polymer, gebildet.

In einer weiteren Ausgestaltung können die Kammern durch Segmentierung eines Schlauches, der sich in der Längsrichtung erstreckt, ausgebildet sein. Die Kammern werden beispielsweise in dem Schlauch dadurch gebildet, dass der Strömungskanal des Schlauches in regelmäßigen/unregelmäßigen Abständen verschlossen wird.

Es ist allerdings auch möglich, dass jede Kammer jeweils durch einen beidseitig verschlossenen Schlauch ausgebildet ist und die Schläuche in der Längsrichtung aufeinanderfolgend angeordnet sind.

Damit der Schlauch bzw. die Schläuche bei Einleiten des Mediums ein gewisses Ausdehnungsverhalten hat/haben, sind der Schlauch bzw. die Schläuche beispielsweise derart dimensioniert, dass der Innendurchmesser des Schlauches nicht kontant ist und sich hierdurch unterschiedliche Wandstärken des Schlauches bzw. der Schläuche ergeben.

Als Material für die Schläuche kann bevorzugt ebenfalls ein elastisches Material, wie beispielsweise ein Elastomer, Silikon, Latex oder allgemein ein elastisches Polymer verwendet werden.

Bevorzugt können die Kammern der erfindungsgemäßen Expansionsvorrichtung jeweils über einen Druckschlauch mit dem Medium, d.h. der Flüssigkeit, dem Gel oder dem Gas, gefüllt werden.

Bevorzugt kann der Druckschlauch zumindest teilweise mit einem Material oder einer Substanz beschichtet sein, das/die eine bakterielle Besiedelung des Druckschlauches verhindert. Bei einer solchen Beschichtung kann es sich beispielsweise um eine Antibiotikum-/Antibiotikaschicht, eine Silberschicht oder silberhaltige Schicht handeln.

Die Ausdehnungseinrichtungen können in einer weiteren Ausgestaltung auch mechanische Elemente sein.

Beispielsweise sind die mechanischen Elemente Federelemente, die mit einer Gewindespindel derart ineinandergreifen, dass durch Drehen der Gewindespindel die Federelemente ausgedehnt werden.

Bevorzugt weisen die mechanischen Federelemente mindestens zwei, drei oder vier Blattfedern auf, die mit der Gewindespindel mittelbar oder unmittelbar ineinandergreifen und durch Drehen der Gewindespindel ausgedehnt werden.

In einer bevorzugten Ausgestaltung weisen die mechanischen Federelemente jeweils mindestens zwei, drei oder vier Blattfedern auf, die an mindestens einer mit der Gewindespindel ineinandergreifenden Hülse befestigt sind, und durch Drehen der Gewindespindel die Hülse derart versetzbar ist, dass sich die Blattfedern ausdehnen.

In einer weiteren bevorzugten Ausgestaltung sind die mechanischen Elemente Scherenelemente, die mit der Gewindespindel derart ineinandergreifen, dass durch Drehen der Gewindespindel die Scherenelemente ausgedehnt werden.

Bevorzugt weist die Expansionsvorrichtung mindestens ein Kraftverteilungselement auf, über das die Ausdehnungseinrichtungen bei bestimmungsgemäßer Verwendung der Expansionsvorrichtung die Kraft auf den Knochen mittelbar ausüben.

Bei einem solchen Kraftverteilungselement handelt es sich beispielsweise um eine Kraftverteilungsstrebe, die von mehreren der mechanischen Elemente getragen wird und die bei bestimmungsgemäßer Verwendung der Expansionsvorrichtung, d.h. bei der Knochenexpansion, zwischen den mechanischen Elementen und dem Knochen angeordnet ist. Hierdurch wird die auf den Knochen ausgeübte Kraft besser verteilt.

Zur Erhöhung der Stabilität der Expansionsvorrichtung kann eine Metallseele in der Längsrichtung durch die Expansionsvorrichtung hindurch verlaufen. Hierdurch lässt sich die erfindungsgemäße Expansionsvorrichtung leichter innerhalb des Röhrenknochens, beispielsweise durch einen intramedullären Zugang hindurch, anordnen. Die Metallseele kann darüber hinaus derart dimensioniert sein, dass sie einen guten Röntgenkontrast erzeugt, wodurch die Lage der erfindungsgemäßen Expansionsvorrichtung nach Einsetzen in den Röhrenknochen gut überprüft werden kann.

Die erfindungsgemäße Expansionsvorrichtung umfasst bevorzugt auf ihrer Außenoberfläche mindestens eine Erhebung, die zur Verhinderung einer Lageänderung der Expansionsvorrichtung innerhalb des Röhrenknochens, d.h. im eingesetzten Zustand, vorgesehen ist. Wenn die Expansionsvorrichtung durch den intramedullären Zugang hindurch in den Röhrenknochen eingesetzt ist, sorgt die mindestens eine Erhebung auf der Außenoberfläche dafür, dass sich die Expansionsvorrichtung innerhalb des Röhrenknochens nicht verdreht und nicht ihre Lage ändert.

Bevorzugt ist die mindestens eine Erhebung als eine noppenartige Erhebung oder als ein sich in der Längsrichtung der Expansionsvorrichtung erstreckender Steg ausgebildet.

Wenn die Ausdehnungseinrichtungen als Kammern ausgebildet sind, verlaufen bevorzugt in der Erhebung die Druckschläuche zu den Kammern.

In Abhängigkeit von der beabsichtigten Verwendung der erfindungsgemäßen Expansionsvorrichtung bzw. in Abhängigkeit von der Länge des Röhrenknochens, in den die Expansionsvorrichtung eingesetzt werden soll, kann die Expansionsvorrichtung in ihrer Länge unterschiedlich ausgebildet sein, d.h. ihre Länge zwischen dem proximalen und distalen Ende kann unterschiedlich sein. Bevorzugte Längen betragen 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 21 cm, 22 cm, 23 cm, 24 cm, 25 cm, 26 cm, 27 cm, 28 cm, 29 cm, 30 cm, 31 cm, 32 cm, 33 cm, 34 cm, 35 cm, 36 cm, 37 cm, 38 cm, 39 cm oder 40 cm und können auch zwischen diesen Werten liegen.

Die erfindungsgemäße Expansionsvorrichtung weist bevorzugt in ihrem maximal ausgedehnten Zustand einen Durchmesser zwischen 1,5 cm und 3 cm auf. In ihrem Grundzustand, d.h. in ihrem kontrahierten bzw. zusammengezogenen Zustand beträgt der Durchmesser bevorzugt 0,2 cm bis 0,4 cm.

Erfindungsgemäß wird eine medizinische Vorrichtung zur Knochenexpansion geschaffen, die eine im Vorhergehenden erläuterte Expansionsvorrichtung und einen Aktuator, der eingerichtet ist, die Ausdehnungseinrichtungen der Expansionsvorrichtung auszudehnen, aufweist.

Wenn die Expansionsvorrichtung die Ausgestaltung der Ausdehnungseinrichtungen als befüllbare Kammern aufweist, ist der Aktuator beispielsweise eine Pumpe, die derart eingerichtet ist, dass sie die Kammern mit einem Medium füllen kann.

Wenn die Expansionsvorrichtung in der Ausgestaltung der Ausdehnungseinrichtungen als mechanische Elemente ausgebildet ist, ist der Aktuator bevorzugt ein elektrischer Motor, der derart eingerichtet ist, dass er die mechanischen Elemente ausdehnen kann. Beispielsweise ist der elektrische Motor mit der im Vorhergehenden erläuterten Gewindespindel verbunden bzw. gekoppelt und kann die Gewindespindel zur Ausdehnung der Ausdehnungseinrichtungen drehen.

Bevorzugt ist der Aktuator derart ausgebildet, dass er während der Knochenexpansion im Körpergewebe angeordnet und betrieben werden kann. Der Aktuator ist in diesem Fall beispielsweise von außen betätigbar, sodass der Körper des Patienten nach Implantation der Expansionsvorrichtung und des Aktuators vollständig geschlossen werden kann. Zur Betätigung des Aktuators weist dieser beispielsweise einen Schalter auf, der durch Druckausübung auf das darüberliegende Gewebe oder durch Anlegen eines magnetischen, elektrischen oder elektromagnetischen Feldes betätigt werden kann.

Dadurch dass bei vollständiger Implantation keine Elemente aus dem Körper des Patienten geführt werden müssen, wird die Gefahr einer Infektion oder von Weichteilirritationen verringert.

Wenn die medizinische Vorrichtung die Expansionsvorrichtung in der Ausgestaltung mit befüllbaren Kammern aufweist, ist bevorzugt zusätzlich ein Reservoir für das Medium, das von der Pumpe in die Expansionsvorrichtung gepumpt wird, vorgesehen. Bevorzugt ist das Reservoir derart ausgestaltet, dass es zusammen mit der Expansionsvorrichtung und dem Aktuator mit in den Körper implantiert werden kann.

Das Reservoir kann in diesem Fall ein mit einer Nadel füllbares Reservoir sein. Hierdurch kann das Reservoir leer implantiert werden und anschließend entsprechend gefüllt und/oder nachgefüllt werden.

Beispielsweise ist das Reservoir eine Kammer, Ballon oder dergleichen, die/der an ein Port-Gefäßzugangssystem angeschlossen ist. Das Reservoir kann durch Anstechen des Port-Gefäßzugangssystems mit dem Medium gefüllt werden. Zum Füllen des Reservoirs wird das Port-Gefäßzugangssystem bevorzugt mit einer Nadel (Huber-Schliff) angestochen und mit dem Medium gefüllt. Vorteilhaft hierbei ist, dass sich das Port-Gefäßzugangssystem nach dem Herausziehen der Nadel wieder automatisch verschließt und somit das Medium innerhalb des Reservoirs verleibt. Wie hieraus ersichtlich ist, kann eine ganz bevorzugte medizinische Vorrichtung geschaffen werden, die vollständig in den Körper zur Knochenexpansion/Kallusdistraktion implantiert werden kann.

Wenn die Pumpe nicht so ausgestaltet ist, dass sie vollständig in den Körper des Patienten implantiert wird, ist die Pumpe beispielsweise eine außerhalb des Körpers des Patienten anzuordnende HPLC-Pumpe, die Förderraten des Mediums im Mikroliterbereich zulässt. Die Verwendung einer HPLC-Pumpe ist auch vorteilhaft, weil sie konstante Förderraten des Mediums unanhängig von dem Druckanstieg innerhalb der Kammern ermöglicht.

Die medizinische Vorrichtung weist eine Steuerungseinrichtung auf, die den Aktuator derart ansteuern kann, dass sich die Ausdehnungseinrichtungen nach dem Prinzip der Kallusdistraktion ausdehnen.

Mit der erfindungsgemäßen medizinischen Vorrichtung lässt sich die Ausdehnung eines Röhrenknochens steuern. Wenn die Expansionsvorrichtung in einen in Längsrichtung gespaltenen Röhrenknochen eingesetzt ist, kann der Aktuator die Ausdehnungseinrichtungen der Expansionsvorrichtung derart ausdehnen, dass der sich an dem gespaltenen Röhrenknochen gebildete Kallus distrahiert und ein Röhrenknochen größeren Durchmessers erhalten wird. Beispielsweise kann der Aktuator über die Steuerungseinrichtung derart angesteuert werden, dass sich die Expansionsvorrichtung gleichmäßig oder ungleichmäßig ausdehnt.

Allgemein kann die Steuerungseinrichtung auch als Regelung ausgestaltet sein, wobei an der Expansionsvorrichtung gemessene Größen, wie Druck, Kraft, Volumen oder Längen als Eingangsgrößen zur Regelung in der Steuerungseinrichtung herangezogen werden können.

Bevorzugt umfasst die medizinische Vorrichtung die Expansionsvorrichtung in der Ausgestaltung der Ausdehnungseinrichtungen als befüllbare Kammern, wobei der Aktuator in diesem Fall eine Pumpe ist, die eingerichtet ist, die Kammern mit einem Medium, wie beispielsweise einer Flüssigkeit, einem Gel oder einem Gas, zu füllen. Die Steuerungseinrichtung kann für die Knochenexpansion die Pumpe derart ansteuern, dass sie die Kammern der Expansionsvorrichtung zur Ausdehnung mit dem Medium füllt.

Wenn der zu expandierende Röhrenknochen an einer bestimmten Stelle stabiler bzw. stärker ausgebildet ist, erhöht die Pumpe den Druck in der entsprechenden Kammer der Expansionsvorrichtung, derart, dass sich diese Kammer in einer bestimmten Weise zusammen mit den anderen Kammern ausdehnt. Die Ausdehnung der Kammern kann beispielsweise dadurch gesteuert werden, dass das Volumen einer/eines in die Kammern geleiteten Flüssigkeit/Gels gemessen/bestimmt wird. Wenn das in die Kammer geleitete Medium kompressibel ist, beispielsweise bei der Verwendung von Luft, ist zusätzlich der in der entsprechenden Kammer herrschende Druck bei der Volumenbestimmung mit zu berücksichtigen.

Die Pumpe wird von der Steuerungseinrichtung derart angesteuert, dass sich die Expansionsvorrichtung nach dem Prinzip der Kallusdistraktion ausdehnt. Beispielsweise wird die Pumpe derart angesteuert, dass der Kallus täglich mit 0,5 bis 0,75 mm expandiert bzw. distrahiert werden würde.

Die Bildung bzw. Distraktion des Kallus kann sonografisch überwacht und die Steuerung der Expansionsvorrichtung entsprechend angepasst werden.

Bevorzugt wird als Medium, das von der Pumpe in die Kammern geleitet wird, eine röntgenkontrastgebende Flüssigkeit oder eine inerte Flüssigkeit verwendet. Im ersteren Fall lässt sich hierdurch nach Einführen der Expansionsvorrichtung in den Röhrenknochen die Lage und der Zustand der Expansionsvorrichtung gut bestimmen, wobei im zweiteren Fall das Risiko, wenn die Expansionsvorrichtung eine undichte Stelle aufweisen sollte, einer Gesundheitsgefährdung des Patienten vermindert werden kann.

Wenn die erfindungsgemäße Expansionsvorrichtung und der Aktuator so ausgestaltet sind, dass sie vollständig in den Körper des Patienten implantiert werden können, ist die Steuerungseinrichtung bevorzugt so eingerichtet, dass sie den Aktuator durch Aufbringen einer Druckkraft auf das über dem Aktuator liegende Gewebe oder durch Anlegen eines magnetischen/elektrischen/elektromagnetischen Feldes ansteuern kann.

Beispielsweise wird hierfür an dem entsprechenden Ort des Körpers des Patienten eine Manschette oder dergleichen angebracht, die mit der Steuerungseinrichtung verbunden ist und wodurch die Steuerungseinrichtung Steuersignale an den Aktuator senden und übermitteln kann.

Zur Beschleunigung der Kallusbildung und Kallusreifung kann bevorzugt eine Ultraschallapplikation zum Einsatz kommen. Bevorzugt wird niederenergetischer Ultraschall verwendet, wobei dieser bevorzugt über das Medium (Flüssigkeit, Gel oder Gas) in die Expansionsvorrichtung eingeleitet werden kann. Hierfür kann beispielsweise ein Ultraschallerzeuger innerhalb der Expansionsvorrichtung und/oder innerhalb der Pumpe zur Einleitung des Ultraschalls in das Medium angeordnet sein. Beispielsweise wird der Ultraschall mit einer Frequenz von 1,5MHz und einer Leistung von 30 mW/cm² gepulst (Pulsfrequenz 1kHz, Signallänge 200µs) appliziert.

In einer bevorzugten Ausgestaltung der erfindungsgemäßen medizinischen Vorrichtung ist die Steuerungseinrichtung derart eingerichtet, dass sie die Ausdehnungseinrichtungen kontinuierlich oder schrittweise ausdehnen kann.

Ganz besonders bevorzugt werden die erfindungsgemäße Expansionsvorrichtung und die medizinische Vorrichtung bestimmungsgemäß zur Behandlung von Defekten an Oberschenkelknochen oder Schienbeinknochen eingesetzt, wobei zur Rekonstruktion des entsprechenden Knochens das Wadenbein (Fibula) mittels der erfindungsgemäßen Expansionsvorrichtung ausgedehnt und anschließend zur Behebung des Defekts mikrovaskulär transferiert wird.

Zu Beginn der Behandlung werden ein intramedullärer Zugang am Fibula-Köpfchen und eine Öffnung des Markraumes geschaffen, wobei anschließend die Fibula in einer Länge von beispielsweise 20 cm gespalten wird (Längsosteotomie). Die erfindungsgemäße Expansionsvorrichtung wird dann durch den Zugang hindurch in die Fibula eingesetzt. Die Ausdehnung der Expansionsvorrichtung beginnt in der Regel am 7. Tag nach der Osteotomie, wobei die Kallusdistraktion mit täglich 0,5 bis 0,75 mm durchgeführt wird.

Allgemein steuert, wie im Vorhergehenden bereits erläutert, die Steuerungseinrichtung die Expansionsvorrichtung derart, dass diese sich nach dem Prinzip der Kallusdistraktion ausdehnt. Das "Prinzip der Kallusdistraktion" unterscheidet sich in vielen Fällen in Abhängigkeit von dem Alter und der Konstitution des Patienten. Beispielsweise bildet sich der Kallus bei Kindern schneller als bei Patienten hohen Alters, weshalb die Steuerungseinrichtung derart verstellbar bzw. programmierbar ist, dass das "Prinzip der Kallusdistraktion" an den jeweiligen Patienten gut angepasst werden kann. Beispielsweise ist die Steuerungseinrichtung so programmiert, dass für verschiedene Fälle vorprogrammierte und vorgefertigte Expansionsverfahren gespeichert sind.

Nach einer gewünschten Distraktion des Kallus kann die Konsolidierung des Kallus über einen weiteren Zeitraum von ca. 30 bis 40 Tagen erfolgen. Insgesamt kann nach einem Zeitraum von ca. 60 Tagen nach Beginn der Distraktion der Transfer des konfektionierten Röhrenknochens in mikrovaskulärer Technik vorgenommen werden.

Durch die erfindungsgemäße Expansionsvorrichtung bzw. medizinische Vorrichtung kann die Gesamtzeitdauer der Kallusdistraktion und die Behandlungsdauer von Röhrenknochendefekten erheblich verringert werden.

Im Folgenden werden bevorzugte Ausführungsformen der Expansionsvorrichtung bzw. der medizinischen Vorrichtung anhand der beigefügten Figuren erläutert.
In **Fig. 1A** ist eine erste bevorzugte Ausführungsform einer erfindungsgemäßen medizinischen Vorrichtung schematisch dargestellt, wobei die medizinische Vorrichtung eine Pumpe und eine mit der Pumpe verbundene, erfindungsgemäße Expansionsvorrichtung aufweist.
In **Fig. 1B** ist eine Variante der erfindungsgemäßen medizinischen Vorrichtung gemäß der ersten bevorzugten Ausführungsform der Erfindung gezeigt, wobei sich die medizinische Vorrichtung von der in Fig. 1A gezeigten dadurch unterscheidet, dass die Expansionsvorrichtung kein Hüllelement aufweist.
**Fig. 1C** zeigt eine medizinischen Vorrichtung gemäß einer zweiten bevorzugten Ausführungsform der Erfindung, wobei sich die medizinische Vorrichtung von den in Fig. 1A und 1B gezeigten dadurch unterscheidet, dass sie eine anders ausgestaltete Expansionsvorrichtung aufweist.
In **Fig. 1D** ist eine Variante der erfindungsgemäßen medizinischen Vorrichtung gemäß der zweiten bevorzugten Ausführungsform der Erfindung gezeigt.
In **Fig. 2A** ist die in Fig. 1A gezeigte Expansionsvorrichtung im Querschnitt schematisch gezeigt, wobei der gezeigte Querschnitt einer ersten Kammer der Expansionsvorrichtung entspricht.
In **Fig. 2B** ist die in Fig. 1B gezeigte Expansionsvorrichtung im Querschnitt schematisch gezeigt, wobei der gezeigte Querschnitt einer ersten Kammer der Expansionsvorrichtung entspricht.
In **Fig. 2C** ist die in Fig. 1C und 1D gezeigte Expansionsvorrichtung im Querschnitt schematisch gezeigt, wobei der gezeigte Querschnitt einer ersten Kammer der Expansionsvorrichtung entspricht.
In **Fig. 3A** und **3B** ist eine dritte bevorzugte Ausführungsform einer erfindungsgemäßen medizinischen Vorrichtung schematisch gezeigt, wobei in Fig. 3A eine erfindungsgemäße Expansionsvorrichtung der medizinischen Vorrichtung sich in einem kontrahierten bzw. zusammengezogenen Zustand und in Fig. 3B in einem ausgedehnten Zustand befindet.
In **Fig. 4** ist die erfindungsgemäße Expansionsvorrichtung gemäß der dritten bevorzugten Ausführungsform der medizinischen Vorrichtung im Querschnitt schematisch gezeigt, wobei die gezeigte Ausdehnungseinrichtung zwei Blattfedern umfasst.
In **Fig. 5** ist die erfindungsgemäße Expansionsvorrichtung gemäß der dritten bevorzugten Ausführungsform der medizinischen Vorrichtung im Querschnitt schematisch gezeigt, wobei die gezeigte Ausdehnungseinrichtung vier Blattfedern umfasst.
**Fig. 6A und 6B** zeigen eine vierte bevorzugte Ausführungsform einer erfindungsgemäßen medizinischen Vorrichtung, wobei sich diese Ausführungsform von der in Fig. 3A und 3B gezeigten dadurch unterscheidet, dass die Ausdehnungseinrichtungen anders ausgestaltet sind.

### (erste Ausführungsform)

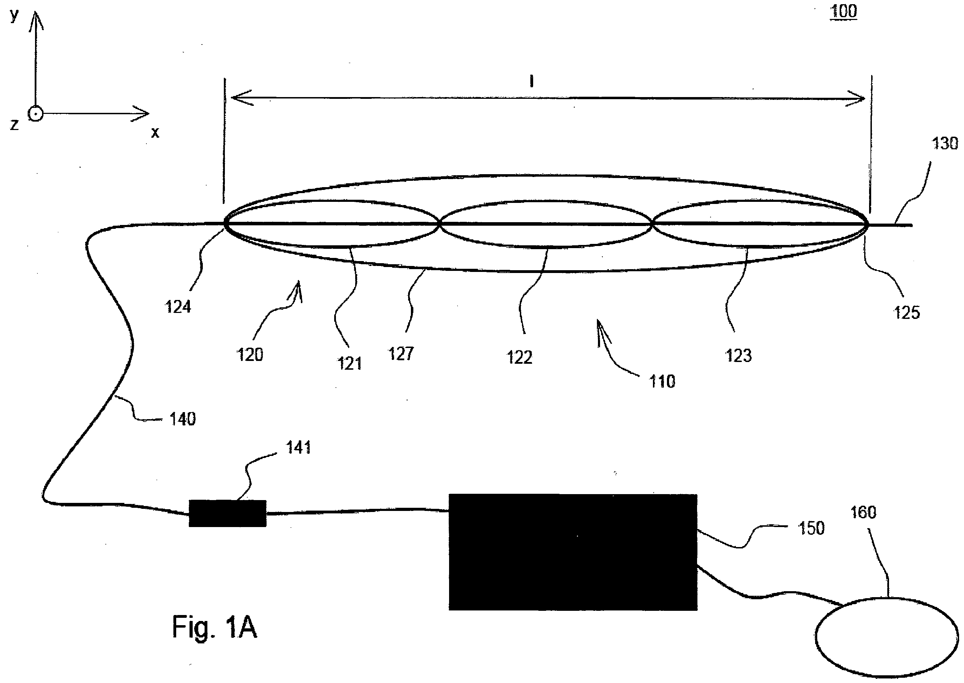

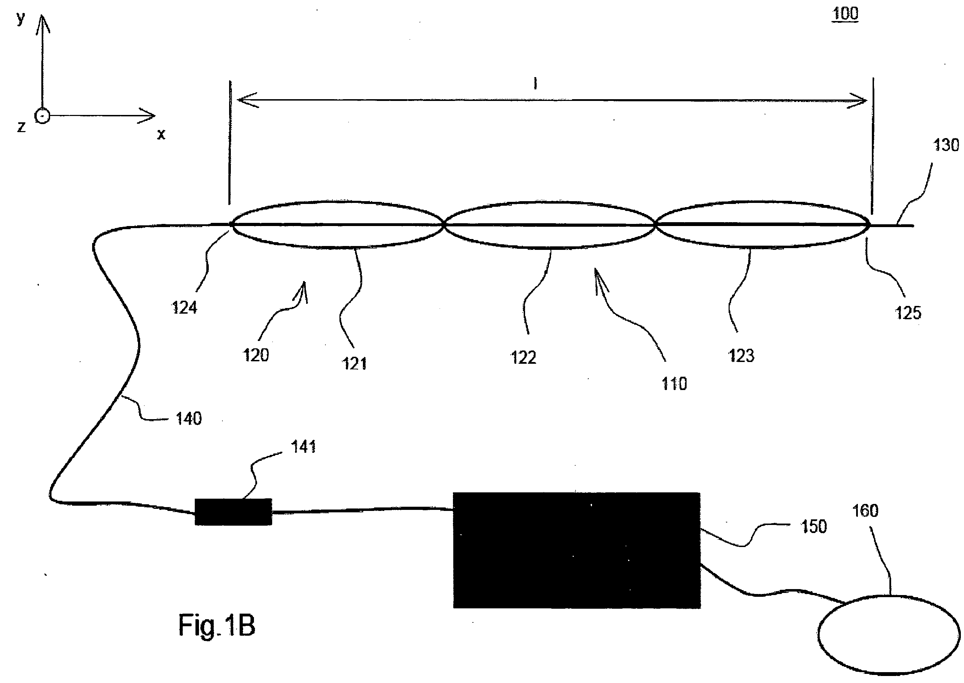

In Fig. 1A und 1B ist eine erste bevorzugte Ausführungsform einer erfindungsgemäßen medizinischen Vorrichtung 100 zur Knochenexpansion schematisch gezeigt.

Die medizinische Vorrichtung 100 umfasst neben der erfindungsgemäßen Expansionsvorrichtung 110 eine Leitung 140 und einen Aktuator 150 in Form einer Pumpe, wobei die Expansionsvorrichtung 110 über die Leitung 140 mit der Pumpe 150 verbunden ist.

Die in Fig. 1A dargestellte Expansionsvorrichtung 110 weist ein als Hüllballon ausgebildetes Hüllelement 120 auf, in dem mehrere Ausdehnungseinrichtungen 121, 122, 123 in Form von Kammern vorgesehen sind.

Das Hüllelement 120 ist aus einem flexiblen Material hergestellt, beispielsweise aus einem polyolefin-basierten Elastomer, einem für medizinische Anwendungen geeigneten Silikon oder allgemein aus einem elastischen Polymer. Das Hüllelement 120 kann sich bei der im Folgenden beschriebenen Ausdehnung zusammen mit den Kammern 121, 122, 123 ausdehnen.

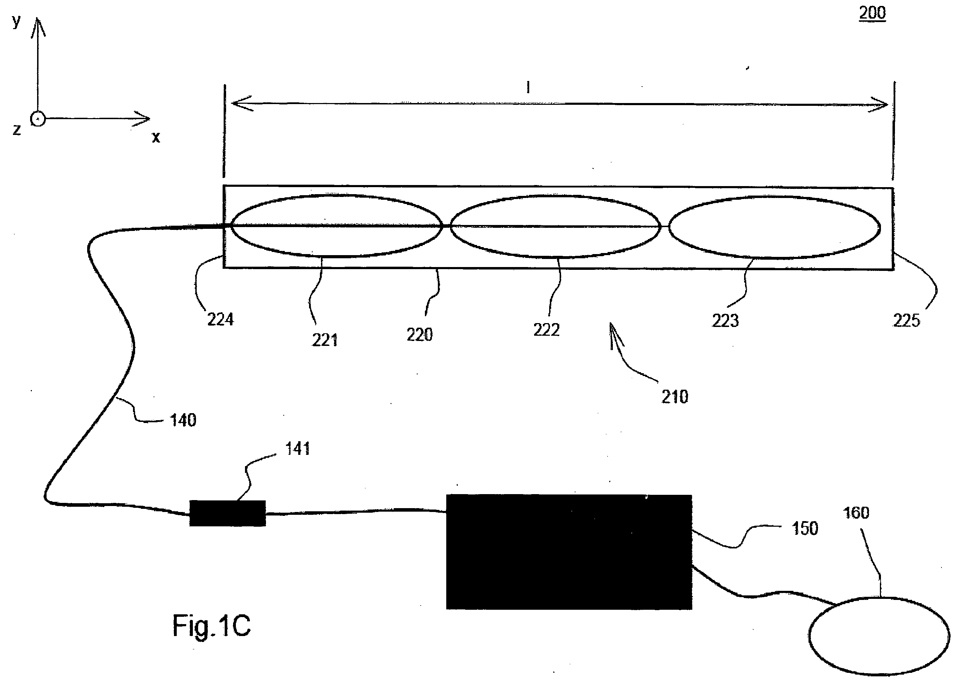

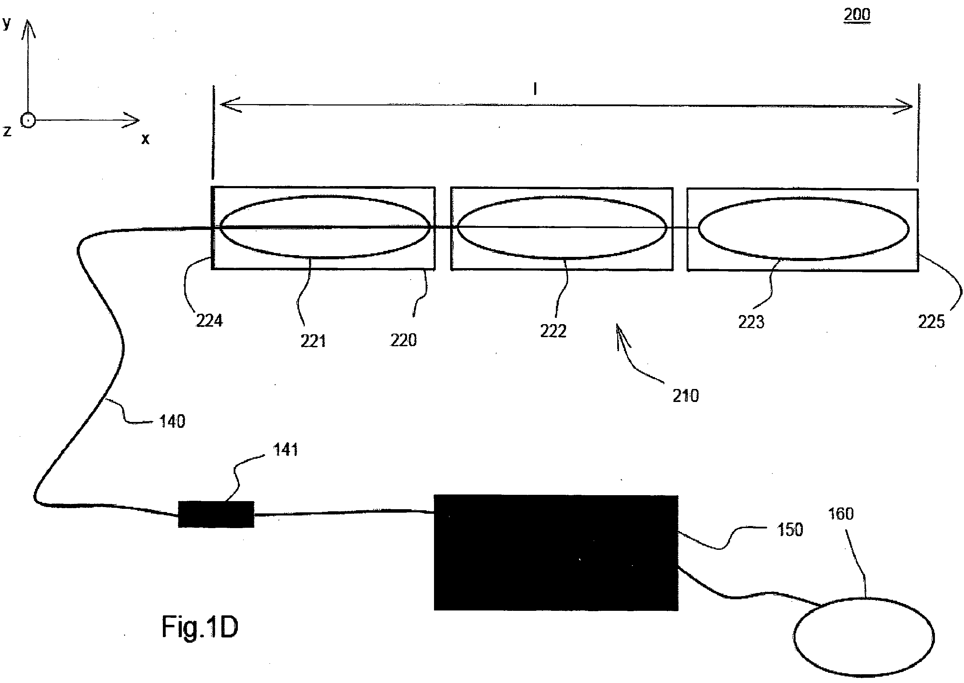

Die in Fig. 1 gezeigte X-Richtung entspricht der Längsrichtung der erfindungsgemäßen Expansionsvorrichtung 110. Die Expansionsvorrichtung 110 erstreckt sich in dieser Längsrichtung zwischen einem proximalen 124 und einem distalen Ende 125.

Das Hüllelement 120 erstreckt sich ebenfalls entlang dieser in Fig. 1A gezeigten X-Richtung und schließt die Kammern 121, 122, 123 vollständig ein.

Innerhalb des Hüllelementes 120 verläuft eine Metallseele 130, die bevorzugt einen Durchmesser von 1 mm aufweist. Die Metallseele 130 ist zur Stabilitätserhöhung der Expansionsvorrichtung 110 vorgesehen und erleichtert die Einführung der Expansionsvorrichtung 110 in einen Röhrenknochen. Bevorzugt ist die Metallseele 130 auch derart ausgebildet, dass sie einen guten Röntgenkontrast aufweist. Hierdurch lässt sich die Lage der Expansionsvorrichtung 110 innerhalb des Röhrenknochens gut überprüfen.

Die gesamte Länge / der in Fig. 1A gezeigten Expansionsvorrichtung 110 liegt bevorzugt zwischen 8 und 25 cm. Ihr max. Durchmesser d beträgt im kontrahierten bzw. zusammengezogenen Zustand 0,3 cm.

Innerhalb des Hüllelementes 120 sind, wie bereits vorher erwähnt, drei Kammern 121, 122, 123 vorgesehen, die jeweils unabhängig und einzeln mit einem bestimmten Medium, wie beispielsweise einer Flüssigkeit, einem Gel oder einem Gas, gefüllt werden können.

Die Kammern 121, 122 und 123 sind in dieser ersten Ausführungsform durch identische Ballons ausgebildet, die in der in Fig. 1A gezeigten Längsrichtung gleichmäßig und direkt aufeinanderfolgend angeordnet sind.

Wenn die Kammern/Ballons 121, 122 und 123 mit dem Medium gefüllt werden, dehnen sich die Ballons 121, 122, 123 in der in Fig. 1A gezeigten Y-Z-Ebene radial aus, wodurch über jede Kammer 121, 122, 123 jeweils eine Kraft auf einen Röhrenknochen ausgeübt werden kann.

Das Hüllelement 120 schließt die Kammern 121, 122 123 bzw. Ballons vollständig und abgedichtet ein. Hierdurch kommen die Kammern 121, 122, 123 mit keinen Körperelementen, wie Knochen, Knochenmark oder Körperflüssigkeiten in direkten Kontakt. Dies hat den Vorteil, dass die Kammern 121, 122, 123 lediglich im Hinblick auf die zu erwartenden maximalen Drücke bzw. Kräfte dimensioniert und materialmäßig ausgebildet werden können.

In dieser ersten bevorzugten Ausführungsform sind die Kammern/Ballons 121, 122, 123 beispielsweise so ausgebildet, dass sie einem Druck von 25bar standhalten können.

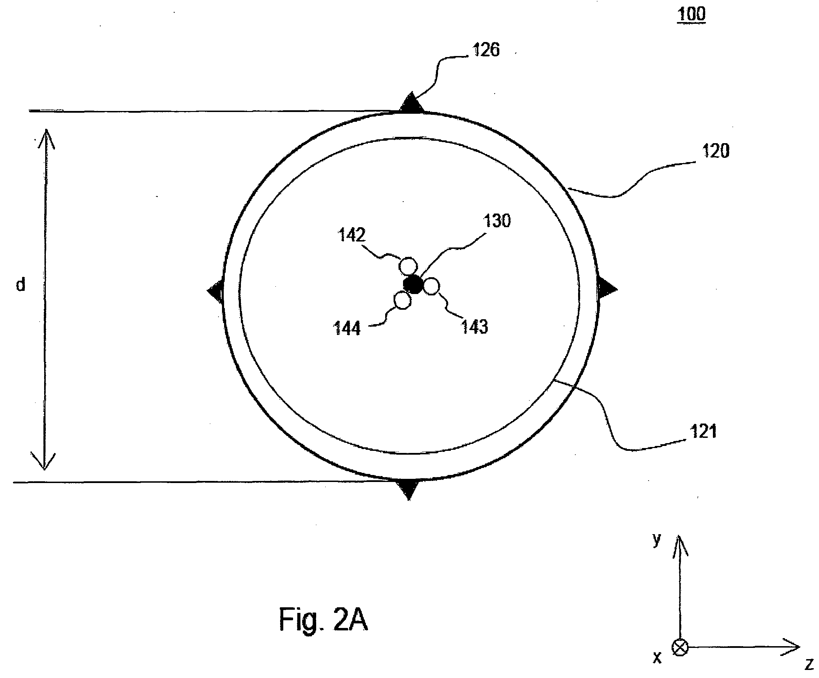

An dem proximalen Ende 124 der Expansionsvorrichtung 110 treten drei Druckschläuche 142, 143, 144 (Fig. 2A), die in der Leitung 140 geführt werden, in die Expansionsvorrichtung 110 ein. Jeder der Druckschläuche 142, 143, 144 mündet jeweils in eine der drei Kammern/Ballons 121, 122, 123.

Über die jeweiligen Druckschläuche 142, 143, 144 kann das Medium in die entsprechende Kammer geleitet werden, wodurch die Expansionsvorrichtung 110 in der in Fig. 1A gezeigten Längsrichtung (X-Richtung) abschnittsweise radial (Y-Z-Ebene) ausgedehnt werden kann. Wie hieraus ersichtlich ist, können die Kammern/Ballons 121, 122, 123 unabhängig voneinander mit dem Medium gefüllt werden, weshalb die Expansionsvorrichtung 110 in einer bestimmten Weise ausgedehnt werden kann.

Die Kammern/Ballons 121, 122, 123 sind in dieser ersten bevorzugten Ausführungsform jeweils mit der Metallseele 130 mittelbar oder unmittelbar (ortsfest) verbunden, sodass sich die Kammern/Ballons 121, 122, 123 beim Einleiten des Mediums hauptsächlich in radialer Richtung (Y-Z-Ebene) ausdehnen.

Die Leitung 140 führt von dem proximalen Ende 124 der Expansionsvorrichtung 110 zu einem Anschlusselement 141 (Konnektions-/Diskonnektionselement), das bei bestimmungsgemäßer Verwendung der medizinischen Vorrichtung 100 innerhalb des Körpergewebes angeordnet ist.

Von dem Anschlusselement 141 verläuft die Leitung 140 weiter zu der Pumpe 150, die sich bei bestimmungsgemäßer Verwendung der medizinischen Vorrichtung außerhalb des Körpers des Patienten befindet.

Dieser Teil der Leitung 140, d.h. von dem Anschlusselement 141 zu der Pumpe 150, ist bevorzugt mit einer Antibiotikabeschichtung und/oder einer Silberbeschichtung zur Vermeidung einer bakteriellen Besiedlung der Leitung 140 beschichtet.

Das Medium, das von der Pumpe 150 in die Kammern/Ballons 121, 122, 123 gepumpt wird, ist in einem Reservoir 160 enthalten. Das Reservoir 160 kann ein Teil der Pumpe 150 oder, wie in Fig. 1A gezeigt, ein separates Reservoir sein.

Alternativ kann die in Fig. 1A gezeigte medizinische Vorrichtung 100 bevorzugt derart ausgestaltet sein, dass die Pumpe 150 und das Reservoir 160 zusammen mit der Expansionsvorrichtung 110 in den Körper des Patienten implantiert werden können. In diesem Fall ist die Expansionsvorrichtung 110, die Leitung 140, die Pumpe 150 und das Reservoir 160 vollständig in dem Körper des Patienten aufgenommen, weshalb keine Leitungen oder Elemente aus dem Körper des Patienten herausgeführt werden müssen und der Körper des Patienten nach der Implantation wieder vollständig verschlossen werden kann. Dies hat den Vorteil, dass Infektionen besser verhindert werden können, da Bakterien und/oder Viren nicht durch eine Eintrittsstelle der Leitung in den Körper des Patienten gelangen können.

Für die Implantation in den Körper ist das Reservoir 160 bevorzugt aus einem flexiblen, elastischen Material gebildet und kann sich zur Aufnahme verschiedener Mengen des Mediums ausdehnen.

Das Volumen des Reservoirs 160 kann in Abhängigkeit davon, welche Menge des Mediums darin aufgenommen werden soll, variieren. Es ist nicht zwingend notwendig, dass das Reservoir 160 bereits gefüllt implantiert wird.

Zum Füllen des Reservoirs 160 kann an diesem ein Port-Gefäßzugangssystem (nicht gezeigt) vorgesehen sein, das in dem Gewebe des Patienten so angeordnet werden kann, dass es mit einer Nadel angestochen und das Medium in das Reservoir 160 geleitet werden kann. Diese Ausgestaltung des Reservoirs 160 ist auch dahingehend vorteilhaft, dass es gut nachgefüllt werden kann.

In Fig. 2A ist die in Fig. 1A gezeigte Expansionsvorrichtung 110 schematisch im Querschnitt gezeigt.

Die in Fig. 2A ersichtliche Kammer entspricht der in Fig. 1A gezeigten Kammer 121, in die eine der Druckschläuche 142, 143, 144 zur Einleitung des Mediums mündet und durch die die verbleibenden Druckschläuche zu den entsprechenden Kammern verlaufen.

Auf dem Hüllelement 120 ist bevorzugt mindestens eine Erhebung 126 - in dieser bevorzugten Ausführungsform vier Erhebungen - ausgebildet, die dafür vorgesehen ist, dass die Expansionsvorrichtung 110 im eingesetzten Zustand ihre Position beibehält. Wenn die erfindungsgemäße Expansionsvorrichtung 110 in den Markraum über einen intramedullären Zugang eingeführt worden ist, und die Expansionsvorrichtung 110 ausgedehnt wird, sorgen die Erhebungen 126 für eine sichere Positionierung der Expansionsvorrichtung 110 innerhalb des Markraumes.

In einer bevorzugten nicht dargestellten Ausbildung der Erhebung(en) 126 können die Druckschläuche 142, 143, 144 darin aufgenommen sein und in der/den Erhebung(en) zu den entsprechenden Kammern/Ballons 121, 122, 123 verlaufen. Diese Ausbildung der Erhebung(en) 126 bzw. diese Verlegung der Druckschläuche 142, 143, 144 hat beispielsweise den Vorteil, dass die Kammern 121, 122, 123 besser an der Metallseele 130 befestigt werden können, da an der Verbindungsstelle zwischen Kammer/Ballon 121, 122, 123 und Metallseele 130 keiner der Druckschläuche 142, 143, 144 hindurchgeführt werden muss.

Die in Fig. 1A dargestellte Pumpe 150 ist dafür vorgesehen, das bestimmte Medium aus dem Reservoir 160 jeweils in die Kammern 121, 122, 123 zu pumpen. Bevorzugt wird als einzuleitendes Medium eine Flüssigkeit, wie zum Beispiel eine Natriumchloridlösung, Wasser oder ein Gel, verwendet.

Die Pumpe 150 wird bevorzugt von einer Steuerungseinrichtung (nicht gezeigt) gesteuert. Die Steuerungseinrichtung kann in der Pumpe 150 mit aufgenommen oder auch eine von der Pumpe 150 getrennte Einheit sein.

Die Steuerungseinrichtung kann die Pumpe 150 derart ansteuern, dass diese das Medium schrittweise in die Kammern pumpt und damit die Expansionsvorrichtung schrittweise ausdehnt. Hierbei kann die Pumpe das Medium in gleichmäßigen Schritten, d.h. immer die gleiche Flüssigkeitsmenge, oder in ungleichmäßigen Schritten, d.h. unterschiedliche Flüssigkeitsmengen, in die Kammern/Ballons 121, 122, 123 leiten. Beispielsweise kann die Steuerungseinrichtung derart programmiert sein, dass sie automatisch in bestimmten Zeitabständen eine definierte Menge des Mediums aus dem Reservoir 160 in die Expansionsvorrichtung 110 pumpt.

Alternativ kann die Steuerungseinrichtung die Pumpe 150 auch derart ansteuern, dass die Pumpe die Expansionsvorrichtung 100 bzw. die Kammern/Ballons 121, 122, 123 von einem Anfangszustand aus kontinuierlich ausdehnt.

Weiterhin alternativ bestimmt die Steuerungseinrichtung beispielsweise anhand der bereits in die Kammern 121, 122, 123 geleiteten Flüssigkeitsmenge das gegenwärtige Volumen der Expansionsvorrichtung 110 bzw. der Kammern/Ballons 121, 122, 123 und die in die Kammern/Ballons 121, 122, 123 einzuleitende Flüssigkeitsmenge, die für eine gewünschte Ausdehnung der Expansionsvorrichtung 110 notwendig ist. Die Steuerungseinrichtung steuert die Pumpe 150 anschließend derart an, dass diese die einzuleitende Flüssigkeitsmenge in die Kammern/Ballons 121, 122, 123 pumpt.

Allgemein ist die Steuerungseinrichtung bevorzugt derart programmiert, dass sie der Expansionsvorrichtung 110 eine solche Menge an Flüssigkeit zuführt, dass sich diese nach dem Prinzip der Kallusdistraktion schrittweise oder kontinuierlich ausdehnt.

Wenn die Pumpe 150, das Reservoir 160 und die Expansionsvorrichtung zusammen in den Körper des Patienten zu implantieren sind, wird die Steuerungseinrichtung bevorzugt so ausgestaltet, dass sie entweder in der Pumpe 150 mit aufgenommen ist oder sie die Pumpe 150 drahtlos ansteuern kann.

Beispielsweise umfasst die Steuerungseinrichtung für die drahtlose Ansteuerung eine Manschette, die an einem bestimmten Körperteil des Patienten angeordnet wird und über die die Steuerungseinrichtung Steuersignale an die implantierte Pumpe 150 übergeben kann, beispielsweise durch Anlegen eines magnetischen, elektrischen oder elektromagnetischen Feldes.

Die Steuerungseinrichtung kann auch als Regelung ausgestaltet sein, wobei eine messtechnisch erfasste Größe, wie der in den Kammern herrschende Druck oder deren Volumen, zur Regelung an die Steuerungseinrichtung zurückgeführt wird.

Dadurch, dass die Expansionsvorrichtung 110 mehrere Kammern/Ballons 121, 122, 123 aufweist, können unterschiedliche Kräfte auf den Röhrenknochen ausgeübt und die Expansionsvorrichtung/der Röhrenknochen in einer bestimmten Weise ausgedehnt werden.

In Fig. 1B ist eine Variante der erfindungsgemäßen medizinischen Vorrichtung 100 gemäß der ersten bevorzugten Ausführungsform der Erfindung gezeigt, wobei sich diese von der in Fig. 1A gezeigten lediglich dadurch unterscheidet, dass die Expansionsvorrichtung 110 kein Hüllelement 127 aufweist. In dieser Variante der Expansionsvorrichtung 110 kommen die Kammern/Ballons 121, 122, 123 in direkten Kontakt mit dem auszudehnenden Knochen, weshalb in dieser Variante die Kammern bzw. die Ballons 121, 122, 123 derart materialmäßig ausgestaltet sind, dass sie sich für den direkten Kontakt mit dem Knochen bzw. Knochenmark eignen.

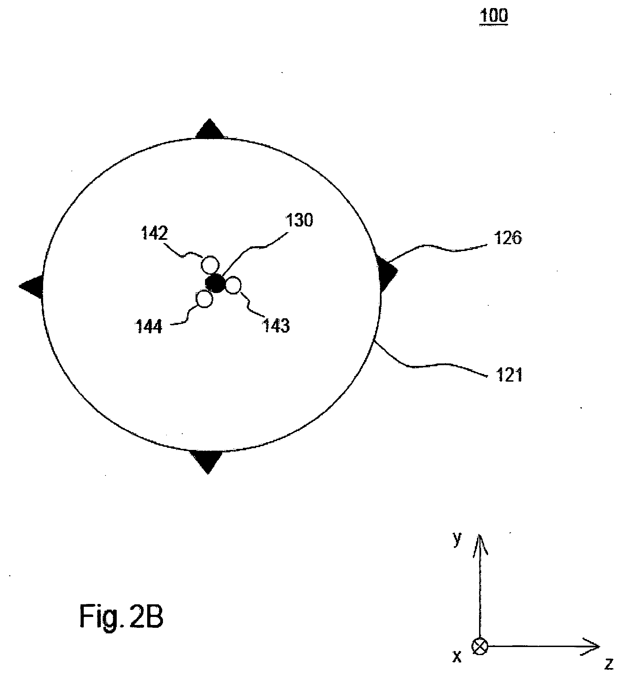

Fig. 2B zeigt schematisch einen Querschnitt der in Fig. 1B gezeigten erfindungsgemäßen Expansionsvorrichtung 100. Der in Fig. 2B gezeigte Querschnitt entspricht einem Querschnitt der ersten Kammer 121. Da diese Variante der erfindungsgemäßen Expansionsvorrichtung 110 kein Hüllelement aufweist, sind die Erhebungen 126 direkt auf den Kammern/Ballons 121, 122, 123 (in Fig. 2B auf der Kammer 121) gebildet.

Die verbleibenden Elemente der in Fig. 1B und 2B gezeigten medizinischen Vorrichtung 100 sind mit denen unter Bezug auf Fig. 1A und 2A beschriebenen identisch, weshalb deren Beschreibung an dieser Stelle nicht wiederholt wird.

### (zweite Ausführungsform)

Fig. 1C zeigt eine zweite bevorzugte Ausführungsform der erfindungsgemäßen medizinischen Vorrichtung. Die in dieser Figur gezeigte medizinische Vorrichtung 200 unterscheidet sich von denen im Vorhergehenden beschriebenen dadurch, dass die Expansionsvorrichtung 210 anders ausgestaltet ist. In dieser zweiten bevorzugten Ausführungsform ist die Expansionsvorrichtung 210 durch einen Schlauch 220 ausgebildet, der in der in Fig. 1C gezeigten Längsrichtung (X-Richtung) unterteilt ist. Für die Segmentierung des Schlauchs kann dieser beispielsweise mit einem Silikon an entsprechenden Stellen gefüllt und derart verschlossen werden, dass die Kammern 221, 222, 223 ausgebildet werden.

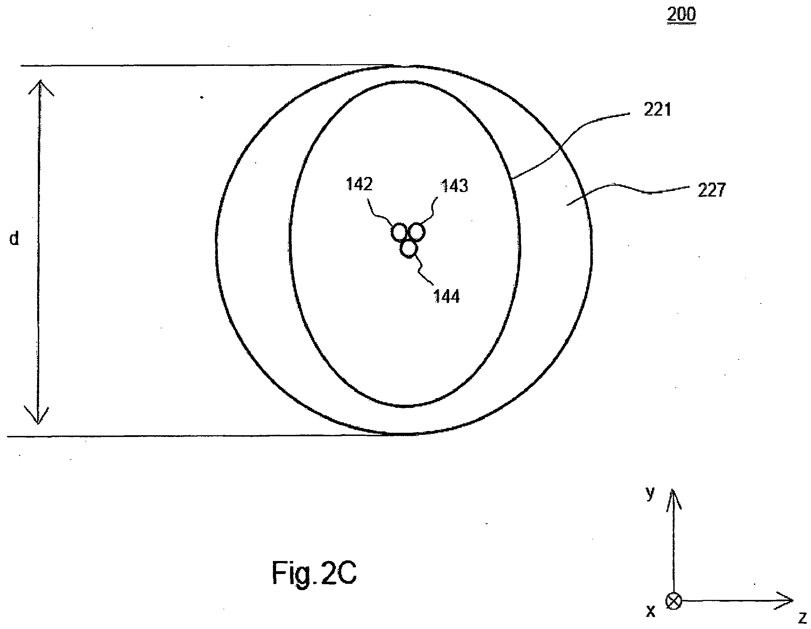

Fig. 2C zeigt einen Querschnitt der in Fig. 1C gezeigten erfindungsgemäßen Expansionsvorrichtung 210. Wie hieraus ersichtlich ist, verlaufen in dieser zweiten Ausführungsform ebenfalls die Druckschläuche 142-144 durch die Kammern 221, 222, 223, wobei jeder der Druckschläuche jeweils in eine zugeordnete Kammer 212, 222, 223 mündet.

Bevorzugt ist der Schlauch 220 so ausgebildet, dass er keinen konstanten Innendurchmesser aufweist, weshalb sich die Wandstärke des Schlauchs 220 ändert. Dies ist dahingehend vorteilhaft, dass der Schlauch 220 sich bei der Ausdehnung nicht in alle Richtungen gleichmäßig ausdehnt, weshalb sich die Expansionsvorrichtung 210 innerhalb des Knochenmarks nicht ohne weiteres verdrehen lässt und ortsfest bleibt.

In Fig. 1D ist eine Variante der erfindungsgemäßen medizinischen Vorrichtung 200 gemäß der zweiten bevorzugten Ausführungsform gezeigt, wobei sich diese von der unter Bezug auf Fig. 1C beschriebenen dadurch unterscheidet, dass der Schlauch 227 in einzelne jeweils einer Kammer zugeordnete Teilabschnitte/Schlauchstücke unterteilt ist. Jedem Schlauchstück ist eine der Kammern 221, 222, 223 zugeordnet.

In allen unter Bezug auf Fig. 1A - 1D gezeigten Ausführungsformen und Varianten der medizinischen Vorrichtung sind die Pumpe 150, die Leitung 140, das Reservoir 160 und die Steuerungseinrichtung identisch, weshalb die diesbezüglichen Erläuterungen für alle Ausführungsformen und Varianten der erfindungsgemäßen medizinischen Vorrichtung gleichermaßen gelten.

### (dritte Ausführungsform)

Im Folgenden wird eine dritte bevorzugte Ausführungsform der medizinischen Vorrichtung zur Knochenexpansion beschrieben.

Die medizinische Vorrichtung 300 umfasst eine Expansionsvorrichtung 310 zur Knochenexpansion.

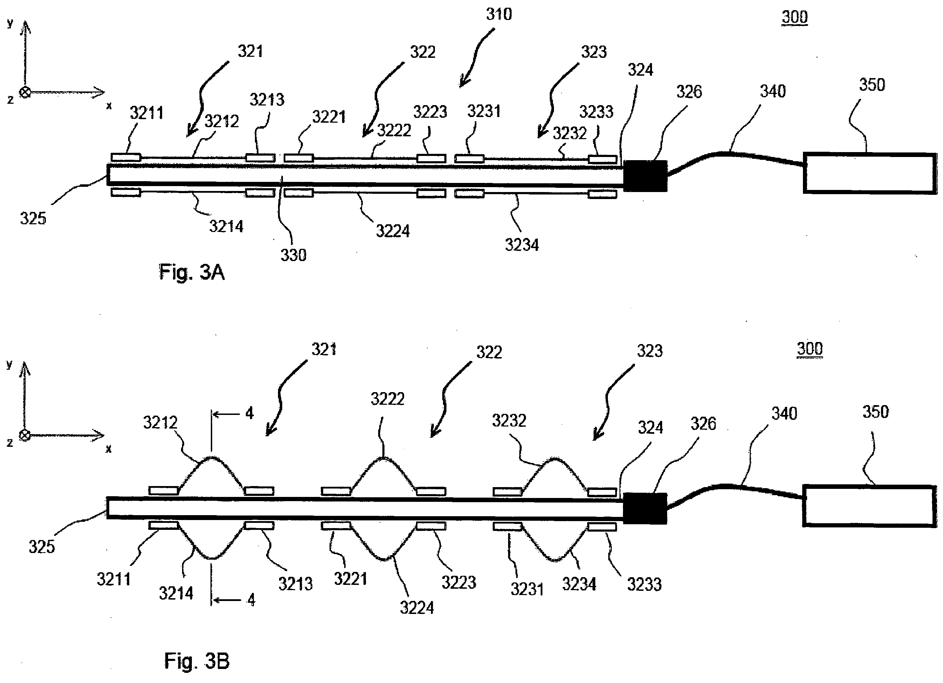

Fig. 3A und 3B zeigen die Expansionsvorrichtung 310 einerseits in einem zusammengezogenen bzw. kontrahierten Zustand (Fig. 3A) und andererseits in ihrem ausgedehnten Zustand (Fig. 3B).

Die in Fig. 3A und 3B gezeigte X-Richtung entspricht der Längsrichtung der erfindungsgemäßen Expansionsvorrichtung 310, in der sich die Expansionsvorrichtung 310 von einem proximalen Ende 324 zu einem distalen Ende 325 erstreckt.

Wie in Fig. 3A gezeigt, umfasst die Expansionsvorrichtung 310 drei Ausdehnungseinrichtungen 321, 322, 323 die in dieser Ausführungsform als mechanische Federelemente ausgebildet sind. Die Federelemente 321, 322, 323 sind identisch aufgebaut.

Die Federelemente 321, 322, 323 weisen jeweils eine erste Blattfeder 3212, 3222, 3232 und eine zweite Blattfeder 3214, 3224, 3234 auf, die jeweils einerseits an einer ersten Hülse 3211, 3221, 3231 und andererseits an einer zweiten Hülse 3213, 3223, 3233 befestigt sind.

Die Federelemente 321, 322, 323 wirken mit einer Gewindespindel 330 derart zusammen, dass die Federelemente 321, 322, 323 bzw. deren Blattfedern ausgedehnt werden können. Hierfür werden die ersten und zweiten Hülsen auf der Gewindespindel 330 angeordnet bzw. die Gewindespindel 330 durch die ersten und zweiten Hülsen hindurchgeführt.

Die ersten Hülsen 3211, 3221, 3231 und die Gewindespindel 330 greifen über ein Gewinde derart ineinander, dass durch Drehen der Gewindespindel 330 die ersten Hülsen 3211, 3221, 3231 auf der Gewindespindel 330 translatorisch in der X-Richtung versetzt werden können. Der Zustand der Expansionsvorrichtung 310, in dem die ersten Hülsen 3211, 3221, 3231 versetzt sind, ist in Fig. 3B gezeigt.

Die zweiten Hülsen 3213, 3223, 3233 sind drehbar auf der Gewindespindel 330 gelagert, werden allerdings nicht durch Drehung der Gewindespindel 330 versetzt, sondern bleiben bei Drehung der Gewindespindel 330 in Bezug auf die Längsrichtung der Expansionsvorrichtung 310 (X-Richtung) ortsfest.

Durch diese Ausbildung der Federelemente 321, 322, 323 werden bei Drehung der Gewindespindel 330 die ersten Hülsen 3211, 3221, 3231 in der in Fig. 3A gezeigten Längsrichtung (X-Richtung) translatorisch versetzt, wodurch sie sich den zweiten Hülsen 3213, 3223, 3233 annähern und hierdurch die ersten und zweiten Blattfedern 3212, 3214, 3222, 3224, 3232, 3234 nach außen (positive und negative Y-Richtung) gebogen werden.

Folglich dehnt sich die Expansionsvorrichtung 310 bzw. dehnen sich die Federelemente 321, 322, 323 bei Drehung der Gewindespindel 330 aus, wobei, wenn die Expansionsvorrichtung 310 innerhalb eines auszudehnenden Röhrenknochens angeordnet ist, über jedes Federelement 321, 322, 323 eine Kraft auf den Röhrenknochen zur Kallusdistraktion ausgeübt wird.

In Abhängigkeit von der Dimensionierung der Federelemente 321, 322, 323, wie beispielsweise der Wahl der Federkonstanten und der Längen der Blattfedern, und dem Grad der Versetzung der ersten Hülsen 3211, 3221, 3231 kann eine bestimmte Kraft über jedes der Federelemente 321, 322, 323 auf den Röhrenknochen zur Kallusdistaktion ausgeübt werden.

Die Gewindespindel 330 ist bevorzugt derart ausgebildet, dass sie sich zu einem gewissen Grad biegen lässt. Hierdurch wird die Anordnung der Expansionsvorrichtung 330 innerhalb eines Röhrenknochens durch einen intramedullären Zugang hindurch erleichtert.

An dem proximalen Ende 324 der erfindungsgemäßen Expansionsvorrichtung 310 ist eine in Fig. 3A und 3B gezeigte Kopplungseinrichtung 326 vorgesehen, an der die Expansionsvorrichtung 310 mit einem Aktuator 350 über ein flexibles Torsionsübertragungselement 340 gekoppelt werden kann.

In dieser dritten bevorzugten Ausführungsform ist der Aktuator durch einen Elektromotor realisiert, der von einer Steuerungseinrichtung (nicht gezeigt) derart betrieben wird, dass er die Gewindespindel 330 zur Ausdehnung der Expansionsvorrichtung 310 dreht.

Wie auch in der ersten und zweiten bevorzugten Ausführungsform kann der Aktuator, d.h. der Elektromotor 350, so ausgestaltet sein, dass dieser zusammen mit der Expansionsvorrichtung 310 in den Körper des Patienten implantiert werden kann.

Die Steuerungseinrichtung kann den Elektromotor derart ansteuern, dass dieser die Gewindespindel 330 schrittweise dreht und damit die Federelemente 321, 322, 323 schrittweise ausdehnt. Hierbei kann der Elektromotor 350 die Gewindespindel 330 in gleichmäßigen Schritten oder in ungleichmäßigen Schritten drehen.

Beispielsweise kann die Steuerungseinrichtung derart programmiert sein, dass sie in bestimmten Zeitabständen die Gewindespindel 330 automatisch um definiertes Maß dreht.

Alternativ kann die Steuerungseinrichtung den Elektromotor 350 auch derart ansteuern, dass die Expansionsvorrichtung 310 bzw. die Federelemente 321, 322, 323 von einem Anfangszustand aus kontinuierlich ausdehnt werden.

Wie auch in den vorhergehenden bevorzugten Ausführungsformen steuert/regelt die Steuerungseinrichtung den Aktuator bzw. den Elektromotor derart, dass er die Expansionsvorrichtung 330 nach dem Prinzip der Kallusdistraktion ausdehnt.

Bevorzugt wird zur Regelung der Ausdehnung der Expansionsvorrichtung 310 die zur Drehung der Gewindespindel 330 notwendige Kraft, die einen Rückschluss auf die von den Federelementen 321, 322, 323 auf den Röhrenknochen ausgeübten Kräfte liefert, ermittelt und zur Regelung herangezogen (bzw. zur Steuerungseinrichtung zurückgeführt).

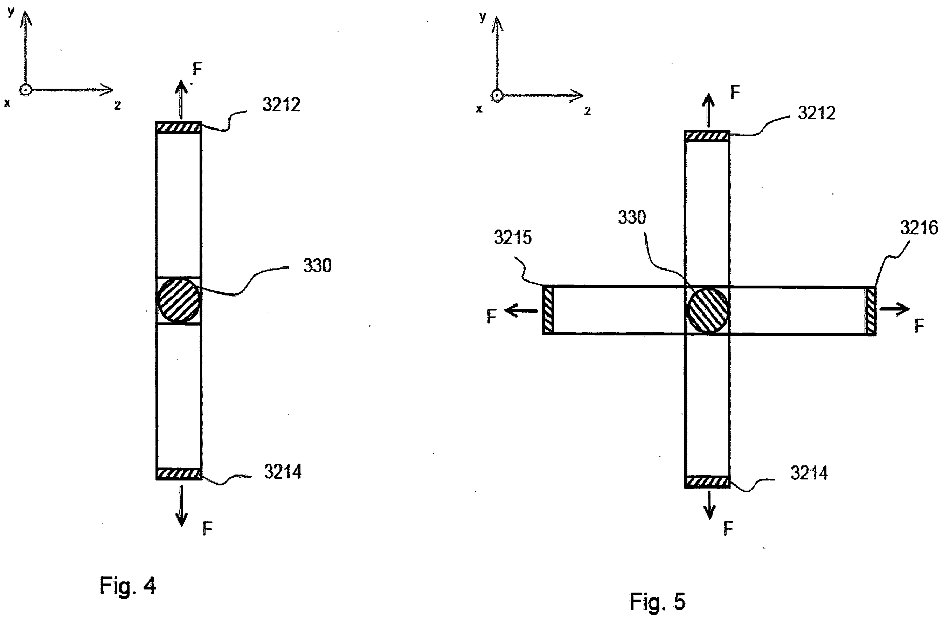

Fig. 4 zeigt eine schematische Schnittansicht der erfindungsgemäßen Expansionsvorrichtung 310 entlang der in Fig. 3B gezeigten Schnittlinie 4-4.

Wie hieraus ersichtlich ist, können auf einen Röhrenknochen, in dem die Expansionsvorrichtung 310 angeordnet ist, über die Blattfedern 3212, 3214, die sich in der gezeigten Y-Richtung ausdehnen, die Kräfte F ausgeübt werden.

Zur Herstellung der Ausdehnungseinrichtungen bzw. der Federelemente 321, 322, 323 werden bevorzugt Hohlprofile (Röhren oder Polygone) verwendet, in die die Gewindespindel 330 eingeführt werden kann. Die Hohlprofile werden an zwei gegenüberliegenden und in Längsrichtung (X-Richtung) verlaufenden Abschnitten derart (partiell) geschlitzt, dass die in Längsrichtung liegenden Endabschnitte der Hohlprofile die erste und zweite Hülse bilden. Die zwischen den Hülsen liegenden und in Längsrichtung verlaufenden Abschnitte bilden die Blattfedern.

Zur Verhinderung einer Überbelastung der Federelemente 321, 322, 323 können bevorzugt an den ersten und/oder zweiten Hülsen Anschläge vorgesehen sein, die in Anschlag kommen, wenn die ersten Hülsen 3211, 3221, 3231 über ein gewisses Maß hinaus versetzt werden. Die Anschläge sind bevorzugt in der in Fig. 4 gezeigten Y-Z-Ebene zwischen den Blattfedern angeordnet. Insoweit können die Anschläge auch aus dem Hohlprofil bei der Herstellung der Federelemente herausgeschnitten werden.

Im Folgenden werden noch bevorzugte Abwandlungen der dritten bevorzugten Ausführungsform der medizinischen Vorrichtung 300 bzw. der Expansionsvorrichtung 310 erläutert.

Im Allgemeinen können, wie in Fig. 5 an dem ersten Federelement 321 exemplarisch gezeigt, die Federelemente 321, 322, 323 auch mehrere Blattfedern, beispielsweise drei oder vier Blattfedern, aufweisen.

Wie aus Fig. 5 ersichtlich ist, sind die dritte und vierte Blattfeder 3215, 3216 relativ zu der ersten und zweiten Blattfeder 3212, 3214 um 90° (um die Achse der Gewindespindel 330) versetzt angeordnet. Die dritte und vierte Blattfeder 3215, 3216 sind ebenfalls an der ersten und zweiten Hülse 3211, 3213 befestigt. Wenn die Gewindespindel 330 gedreht wird, werden die dritte und vierte Blattfeder 3215, 3216 gleichermaßen wie die erste und zweite Blattfeder 3212, 3214 nach außen gebogen bzw. das entsprechende Federelement 321 ausgedehnt.

Die Federelemente 321, 322, 323 sind im Vorhergehenden als identisch beschrieben worden. Die Erfindung ist allerdings hierauf nicht beschränkt. Die Federelemente 321, 322, 323 können auch unterschiedlich sein. Beispielsweise können die Federkonstanten der einem Federelement oder unterschiedlichen Federelementen zugeordneten Blattfedern unterschiedlich sein.

Außerdem können die Federelemente 321, 322, 323 derart ausgestaltet sein, dass auch die entsprechenden zweiten Hülsen 3213, 3223, 3233 über ein Gewinde mit der Gewindespindel 330 ineinandergreifen. In diesem Fall ist es notwendig, dass die Gewindespindel 330 in dem Teilbereich, der einem der Federelemente zugeordnet ist, derart gegenläufige Gewinde aufweist, dass in Abhängigkeit von der Drehrichtung der Gewindespindel 330 die jeweiligen ersten und zweiten Hülsen sich entweder annähern oder in entgegengesetzter Richtung versetzt werden.

Wie aus der vorhergehenden Beschreibung der dritten bevorzugten Ausführungsform ersichtlich ist, können die auszudehnenden Federelemente 321, 322, 323 in Abhängigkeit von der Art des auszudehnenden Röhrenknochens einheitlich oder unterschiedlich dimensioniert werden, so dass der Röhrenknochen, in dem die Expansionsvorrichtung 310 angeordnet ist, in einer bestimmten Art und Weise - bevorzugt nach dem Prinzip der Kallusdistraktion - ausgedehnt werden kann.

### (vierte Ausführungsform)

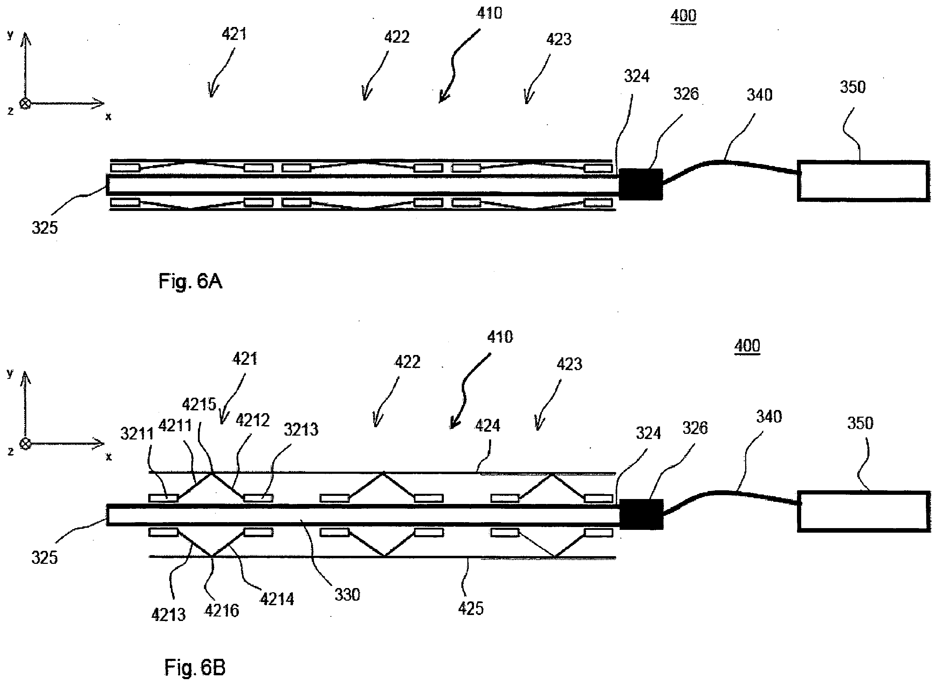

Fig. 6A und 6B zeigen eine vierte Ausführungsform der erfindungsgemäßen medizinischen Vorrichtung.

Die Elemente der vierten bevorzugten Ausführungsform der medizinischen Vorrichtung 400, die mit denen der dritten bevorzugten Ausführungsform identisch sind, weisen das gleiche Bezugszeichen auf und werden nicht nochmals erläutert.

Die medizinische Vorrichtung 400 unterscheidet sich von der unter Bezug auf Fig. 3A und 3B erläuterten dadurch, dass die Ausdehnungseinrichtungen der Expansionsvorrichtung 410 keine Federelemente, sondern als Scherenelemente 421, 422, 423 ausgebildet sind.

Die Expansionsvorrichtung 410 weist ebenfalls drei Ausdehnungseinrichtungen 421, 422, 423 auf, wobei anstatt der Blattfedern die entsprechenden Hülsen 3211, 3213, 3221, 3223, 3231, 3233 über Streben miteinander verbunden sind. Die Ausdehnungseinrichtungen, d.h. die Scherenelemente 421, 422, 423, sind identisch aufgebaut, weshalb diese lediglich unter Bezug auf das erste Scherenelement 421 beschrieben werden.

Die Streben 4211, 4212, 4213, 4214 des ersten Scherenelementes 421 sind einerseits an den Hülsen 3211, 3213 drehbar gelagert befestigt und andererseits an einer gemeinsamen Verbindungsstelle 4215, 4216 drehbar gelagert miteinander verbunden.

Wenn die Gewindespindel 330 gedreht wird, führt dies zu einer Versetzung der Hülse 3211, wodurch die drehbar gelagerten Streben 4211, 4212, 4213, 4214 nach außen (in Fig. 6A und 6B in der Y-Richtung) ausgelenkt werden.

Die Streben 4211, 4212, 4213, 4214 tragen an ihren gemeinsamen Verbindungsstellen 4215, 4216 jeweils ein Kraftverteilungselement 424, 425, über das das Scherenelement 421 bei Drehung der Gewindespindel 330 die Kraft zur Knochenexpansion bzw. zur Kallusdistraktion auf den Knochen ausüben kann, wobei die Kraftverteilungselemente 424, 425 so ausgestaltet sind, dass sie die ausgeübte Kraft flächenmäßig verteilen.

Die Kraftverteilungselemente 424, 425 sind in dieser Ausführungsform als Kraftverteilungsstreben ausgebildet, die jeweils in der Längsrichtung (X-Richtung) verlaufen und von den in Längsrichtung aufeinanderfolgenden Scherenelementen 421, 422, 423 getragen/gehaltert werden. Die Kraftverteilungsstreben 424, 425 werden hierbei jeweils an den Verbindungsstellen der Streben des ersten bis dritten Scherenelementes befestigt, sodass die Kraftverteilungsstreben 424, 425 bei Drehung der Gewindespindel 330 und Ausdehnung der Scherenelemente 421, 422, 423 gleichmäßig angehoben werden.

In Fig. 6A und 6B sind die Scherenelemente 421, 422, 423 so ausgebildet, dass sie sich wie die in Fig. 4 gezeigten Blattfedern in der Y-Richtung ausdehnen.

Allerdings können die Scherenelemente 421, 422, 423 auch zusätzliche Streben aufweisen, die wie auch die in Fig. 5 gezeigten Blattfedern um 90° versetzt sind. Hierdurch kann die zur Knochenexpansion auf den Knochen ausgeübte Kraft besser verteilt werden.

Die zusätzlichen Streben können ebenfalls an ihren Verbindungsstellen ein Kraftverteilungselement bzw. eine Kraftverteilungsstrebe tragen.

Wenn die Kraftverteilungselemente als die gezeigten Kraftverteilungsstreben 424, 425 ausgebildet sind, haben die Kraftverteilungsstreben 424, 425 bevorzugt einen derart kreisbogenförmigen Querschnitt (Y-Z-Ebene), dass die Kraftverteilungsstreben 424, 425 in dem vollständig kontrahierten Zustand der Expansionsvorrichtung 410 eine im Querschnitt geschlossene Hülle ausbilden.

Wie aus den Beschreibungen der erfindungsgemäßen medizinischen Vorrichtungen ersichtlich wird, können Vorrichtungen für eine schnellere Knochenexpansion/Kallusdistraktion zur Verfügung gestellt werden.

## Patentansprüche

1. Medizinische Vorrichtung aufweisend
eine Expansionsvorrichtung zur Knochenexpansion, welche ein proximales Ende und ein distales Ende, zwischen denen sich die Expansionsvorrichtung in einer Längsrichtung erstreckt, und Ausdehnungseinrichtungen, die in der Längsrichtung aufeinanderfolgend angeordnet sind, aufweist, wobei
die Ausdehnungseinrichtungen zur Kraftausübung auf einen Knochen jeweils radial ausgedehnt werden können;
einen Aktuator, der eingerichtet ist, die Ausdehnungseinrichtungen der Expansionsvorrichtung auszudehnen; **gekennzeichnet durch**
eine Steuerungseinrichtung, die den Aktuator derart ansteuern kann, dass sich die Ausdehnungseinrichtungen nach dem Prinzip der Kallusdistraktion ausdehnen

2. Medizinische Vorrichtung gemäß Anspruch 1, wobei
die Steuerungseinrichtung den Aktuator derart ansteuern kann, dass sich die Ausdehnungseinrichtungen gleichmäßig oder ungleichmäßig ausdehnen.

3. Medizinische Vorrichtung gemäß Anspruch 1 oder 2, wobei die Ausdehnungseinrichtungen Kammern sind, die mit einem Medium gefüllt werden können und der Aktuator eine Pumpe ist, die derart eingerichtet ist, dass sie die Kammern mit dem Medium füllen kann.

4. Medizinische Vorrichtung gemäß Anspruch 3, wobei
die Pumpe eingerichtet ist, die Kammern mit einer Flüssigkeit zu füllen; und
die Steuerungseinrichtung die Pumpe derart ansteuern kann, dass die Kammern sich nach dem Prinzip der Kallusdistraktion ausdehnen.

5. Medizinische Vorrichtung gemäß Anspruch 4, wobei die Flüssigkeit eine röntgenkontrastgebende Flüssigkeit oder eine inerte Flüssigkeit ist.

6. Medizinische Vorrichtung gemäß einem der Ansprüche 4 oder 5, weiterhin aufweisend einen Ultraschallerzeuger, der derart eingerichtet ist, dass Ultraschall über die Flüssigkeit in die Expansionsvorrichtung eingeleitet werden kann.

7. Medizinische Vorrichtung gemäß einem der Ansprüche 1 bis 6, wobei die Steuerungseinrichtung derart eingerichtet ist, dass sie die Ausdehnungseinrichtungen kontinuierlich oder schrittweise ausdehnen kann.

8. Medizinische Vorrichtung gemäß einem der Ansprüche 1 bis 7, wobei der Aktuator derart ausgebildet ist, dass er während der Knochenexpansion im Körpergewebe angeordnet und betrieben werden kann.

9. Medizinische Vorrichtung gemäß einem der Ansprüche 3 bis 6, wobei die Kammern durch Ballons ausgebildet sind.

10. Medizinische Vorrichtung gemäß einem der Ansprüche 3 bis 6, wobei die Kammern durch Segmentierung eines Schlauches, der sich in der Längsrichtung erstreckt, ausgebildet sind.

11. Medizinische Vorrichtung gemäß einem der Ansprüche 3 bis 6, wobei die Kammern jeweils durch einen Schlauch ausgebildet sind und die Schläuche in der Längsrichtung aufeinanderfolgend angeordnet sind.

12. Medizinische Vorrichtung gemäß einem der Ansprüche 3 bis 6 oder 9 bis 11, wobei die Kammern jeweils über einen Druckschlauch mit dem Medium gefüllt werden können.

13. Medizinische Vorrichtung gemäß Anspruch 12, wobei der Druckschlauch mit einer Schicht beschichtet ist, die eine bakterielle Besiedelung des Druckschlauches verhindert.

14. Medizinische Vorrichtung gemäß einem der Ansprüche 1 bis 13, wobei eine Metallseele in der Längsrichtung durch die Expansionsvorrichtung zur Erhöhung der Stabilität der Expansionsvorrichtung verläuft.

## Claims

1. A medical apparatus comprising:
an expansion device for bone expansion, which comprises a proximal end and a distal end between which the expansion device extends in a longitudinal direction, and expansion means which are arranged in succession in the longitudinal direction, wherein
the expansion means can be expanded radially for exertion of force on a bone;
an actuator which is configured to expand the expansion means of the expansion device; **characterized by**
a controller which can control the actuator such that the expansion means expand in accordance with the principle of callus distraction.

2. The medical apparatus according to claim 1, wherein the controller can control the actuator such that the expansion means expand uniformly or non-uniformly.

3. The medical apparatus according to claim 1 or 2, wherein the expansion means are chambers which can be filled with a medium and the actuator is a pump which is set up such that it can fill the chambers with the medium.

4. The medical apparatus according to claim 3, wherein the pump is set up to fill the chambers with a liquid, and
the controller can control the pump such that the chambers expand in accordance with the principle of callus distraction.

5. The medical apparatus according to claim 4, wherein the liquid is a liquid which provides X-ray contrast or is an inert liquid.

6. The medical apparatus according to claim 4 or 5, furthermore comprising an ultrasound generator which is set up such that ultrasound can be introduced via the liquid into the expansion device.

7. The medical apparatus according to any one of claims 1 to 6, wherein the controller is set up such that it can expand the expansion means continuously or stepwise.

8. The medical apparatus according to any one of claims 1 to 7, wherein the actuator is constructed such that it can be arranged and operated in the body tissue during the bone expansion.

9. The medical apparatus according to any one of claims 3 to 6, wherein the chambers are formed by balloons.

10. The medical apparatus according to any one of claims 3 to 6, wherein the chambers are constructed by segmenting a tube which extends in the longitudinal direction.

11. The medical apparatus according to any one of claims 3 to 6, wherein the chambers are each formed by a tube and the tubes are arranged in succession in the longitudinal direction.

12. The medical apparatus according to any one of claims 3 to 6 or claims 9 to 11, wherein the chambers can respectively be filled with the medium via a pressure tube.

13. The medical apparatus according to claim 12, wherein the pressure tube is coated with a layer which prevents bacterial colonization of the pressure tube.

14. The medical apparatus according to any one of claims 1 to 13, wherein a metal core runs in the longitudinal direction through the expansion device to increase the stability of the expansion device.

## Revendications

1. Dispositif médical, comprenant
un dispositif d'expansion pour l'expansion osseuse, présentant une extrémité proximale et une extrémité distale entre lesquelles s'étend le dispositif d'expansion dans une direction longitudinale, et des dispositifs d'allongement disposés successivement dans la direction longitudinale, où
les dispositifs d'allongement peuvent être étirés pour exercer une force sur un os ;
un actionneur prévu pour étirer les dispositifs d'allongement du dispositif d'expansion ;
**caractérisé par**
un dispositif de commande pouvant commander l'actionneur de sorte que les dispositifs d'allongement s'étirent conformément au principe de distraction osseuse par callotase.

2. Dispositif médical selon la revendication 1, où
le dispositif de commande peut commander l'actionneur de sorte que les dispositifs d'allongement s'étirent de manière régulière ou irrégulière.

3. Dispositif médical selon la revendication 1 ou 2, où
les dispositifs d'allongement sont des chambres pouvant être remplies d'un milieu et où l'actionneur est une pompe prévue pour pouvoir refouler le milieu dans les chambres.

4. Dispositif médical selon la revendication 3, où
la pompe est prévue pour refouler un liquide dans les chambres ; et où
le dispositif de commande peut commander la pompe de sorte que les chambres s'étirent conformément au principe de distraction osseuse par callotase.

5. Dispositif médical selon la revendication 4, où le liquide est un liquide de contraste radio-opaque ou un liquide inerte.

6. Dispositif médical selon la revendication 4 ou 5, comprenant en outre un générateur d'ultrasons, prévu de manière à permettre la pénétration d'ultrasons dans le dispositif d'expansion au moyen du liquide.

7. Dispositif médical selon l'une des revendications 1 à 6, où le dispositif de commande est prévu pour étirer les dispositifs d'allongement de manière continue ou par pas.

8. Dispositif médical selon l'une des revendications 1 à 7, où l'actionneur est réalisé de manière à pouvoir être disposé et mis en service dans le tissu corporel pendant la distraction osseuse.

9. Dispositif médical selon l'une des revendications 3 à 6, où les chambres sont formées par des ballonnets.

10. Dispositif médical selon l'une des revendications 3 à 6, où les chambres sont formées par segmentation d'un tuyau qui s'étend dans la direction longitudinale.

11. Dispositif médical selon l'une des revendications 3 à 6, où les chambres sont formées chacune par un tuyau et où les tuyaux sont disposés successivement dans la direction longitudinale.

12. Dispositif médical selon l'une des revendications 3 à 6 ou 9 à 11, où les chambres peuvent être remplies chacune de milieu au moyen d'un tuyau de refoulement.

13. Dispositif médical selon la revendication 12, où le tuyau de refoulement est revêtu d'une couche qui empêche une colonisation bactérienne du tuyau de refoulement.

14. Dispositif médical selon l'une des revendications 1 à 13, où une âme métallique s'étend au travers du dispositif d'expansion dans la direction longitudinale pour accroître la stabilité du dispositif d'expansion.
